# EUROPÄISCHE PATENTSCHRIFT

(11) **EP 0 565 970 B1**
(45) Veröffentlichungstag und Bekanntmachung des Hinweises auf die Patenterteilung: **24.09.1997**
(21) Anmeldenummer: 93105481.1
(22) Anmeldetag: 02.04.1993
(51) Int. Cl.: A61B 5/14

(54) **Blutlanzettenvorrichtung zur Entnahme von Blut für Diagnosezwecke**
Blood lancet device for obtaining blood samples for diagnosis purpose
Dispositif pour le prélèvement de sang destiné à un but diagnostique

(30) Priorität: 13.04.1992 DE 4212315
(43) Veröffentlichungstag der Anmeldung: 20.10.1993
(60) Teilanmeldung: 97104145.4
(73) Patentinhaber: BOEHRINGER MANNHEIM GMBH, 68305 Mannheim (DE)
(72) Erfinder: Lange, Hans, W-6840 Lampertheim (DE); Böcker, Dirk, Dr.Dr., W-6900 Heidelberg (DE); Edelmann, Hermann, W-8132 Tutzing-Unterz (DE); Rüdinger, Wolfgang, Dr.Dr., W-6943 Birkenau (DE); Argauer, Herbert, W-8481 Pirk (DE)
(74) Vertreter: Pfeifer, Hans-Peter, Dr., Dr. H.-P. Pfeifer Dr. P. Jany, Patentanwälte

(56) Entgegenhaltungen:
- EP-A- 0 254 203
- US-A- 4 924 879

## Beschreibung

Die Erfindung betrifft ein Blutentnahmegerät zur Entnahme von Blut für Diagnosezwecke, welche ein Gehäuse mit einer Austrittsöffnung für die Spitze einer Lanzette, einen in dem Gehäuse entlang einem vorbestimmten geraden Einstichweg beweglichen Lanzettenhalter zur Halterung der Lanzette und eine Lanzettenführung zur Führung des Lanzettenhalters auf dem vorbestimmten geraden Einstichweg aufweist. Der Lanzettenhalter wird bei der Einstich- und Rückführbewegung von einem Lanzettenantrieb bewegt, der ein elastisches Antriebselement, üblicherweise eine Metallfeder aufweist. Er ist in einer ersten Position, in der sich das elastische Antriebselement in einem gespannten Zustand befindet, mittels einer Arretiervorrichtung arretierbar. Nach dem Lösen der Arretiervorrichtung entspannt sich das elastische Antriebselement und seine Bewegung wird durch den Lanzettenantrieb in die Einstichbewegung umgesetzt, wobei die von dem Lanzettenhalter gehaltene Lanzette mit hoher Geschwindigkeit entlang dem vorbestimmten Einstichweg in Einstichrichtung bewegt wird, bis ihre Spitze aus der Austrittsöffnung austritt, um in einem gegen die Austrittsöffnung gedrückten Körperteil (Finger oder Ohrläppchen) eine Wunde zu erzeugen. Unmittelbar danach wird die Lanzette durch den Lanzettenantrieb wieder zurückgeführt.

Weiter richtet sich die Erfindung auf ein Blutentnahmesystem, welches ein Blutentnahmegerät und darauf abgestimmte Lanzetten umfaßt sowie auf ein Verfahren zur Blutentnahme mittels eines solchen Systems.

Um für Diagnosezwecke eine geringe Menge Blut aus dem Finger oder dem Ohrläppchen zu entnehmen, werden in der ärztlichen Praxis Lanzetten verwendet, die manuell oder mit einer einfachen Apparatur von dem Arzt oder Laborpersonal in das entsprechende Körperteil gestochen werden. Die Lanzette muß selbstverständlich scharf und steril sein. Im übrigen sind aber in der ärztlichen Praxis keine besonders hohen Anforderungen zu stellen, weil die Blutentnahmen bei einzelnen Patienten in großen zeitlichen Abständen durchgeführt werden und der Einstich durch trainiertes, speziell ausgebildetes Personal ausgeführt wird.

Wesentlich höher sind dagegen die Anforderungen bei Blutlanzettenvorrichtungen, die zur Handhabung durch den Patienten selbst bestimmt sind. Sie werden vor allem benötigt, um im Rahmen des sogenannten "home monitoring" besonders gefährdeten Patientengruppen eine regelmäßige Überwachung bestimmter analytischer Werte ihres Blutes zu ermöglichen.

Dies gilt insbesondere für Diabetiker, die ihren Blutzuckerspiegel häufig und regelmäßig kontrollieren sollten, um durch Anpassung der Insulininjektionen an den Bedarf, der von der Nahrungsaufnahme, der körperlichen Aktivität und anderen Faktoren abhängt, möglichst ständig innerhalb bestimmter Sollgrenzen zu halten. Dies ist für die Gesundheit solcher Patienten und die Vermeidung schwerwiegender Spätschäden, wie beispielsweise Erblindung und Amputation von Körperteilen, von allergrößter Bedeutung.

Aus diesem Grunde wurden kleine, einfach zu bedienende und verhältnismäßig kostengünstige Analysesysteme entwickelt, die meist aus Blutteststreifen und einem zugehörigen Auswertegerät bestehen. Obwohl deswegen heute die Möglichkeit zur Analyse jedem Patienten problemlos und relativ kostengünstig zur Verfügung gestellt werden kann, hat die Selbstkontrolle der Blutzuckerwerte noch nicht die wünschenswerte allgemeine Verbreitung unter Diabetikern gefunden. Ein Hauptgrund dafür sind die Schmerzen, die mit der Erzeugung der für die Blutentnahme erforderlichen Stichwunde verbunden sind.

Es wurden zahlreiche verschiedene Blutlanzettenvorrichtungen entwickelt, die geeignet sein sollen, die für die Blutgewinnung erforderliche Stichwunde auf einfache Weise und verhältnismäßig schmerzarm zu erzeugen. Beispiele sind in den US-Patentschriften 4 442 836, 4 469 110, 4 535 769 und 4 924 879 beschrieben. Blutentnahmegerät und Lanzette sind meist aufeinander abgestimmt und werden auch als Blutentnahmesystem bezeichnet. Eine Besonderheit des in der US-Patentschrift 4 924 879 beschriebenen Blutentnahmegerätes besteht darin, daß deren Lanzettenantrieb ein Pleuelstangengetriebe aufweist, durch das die Drehbewegung einer Antriebsscheibe in eine Translationsbewegung des Lanzettenhalters übertragen wird. Trotz einiger Fortschritte ist auch bei diesen bekannten, insbesondere für die Benutzung durch den Patienten selbst bestimmten Blutlanzettenvorrichtung der durch den Einstich erzeugte Schmerz noch zu groß.

Der Erfindung liegt daher die Aufgabe zugrunde, bei einfacher Bauweise des Blutentnahmegerätes den Einstich in einer Art und Weise zu erzeugen, die zu einem verminderten Schmerzempfinden führt.

Die Aufgabe wird durch ein Blutentnahmegerät gemäß Anspruch 1 gelöst, wobei dieses insbesondere im Rahmen eines Blutentnahmesystems gemäß Anspruch 9 eingesetzt und in einem Verfahren gemäß Anspruch 17 verwendet wird.

Der Begriff "Getriebe" wird hier im allgemeinen Sinn verstanden, d.h. als eine kinematische Vorrichtung, die zur Kopplung und Umwandlung von Bewegungen dient, wobei im vorliegenden Falle die Bewegung bei der Entspannung des elastischen Antriebselementes (welches nachfolgend ohne Beschränkung der Allgemeinheit auch als Antriebsfeder bezeichnet wird) in die Bewegung des Lanzettenhalters bzw. einer darin, vorzugsweise auswechselbar, befestigten Lanzette umgewandelt wird.

Vorzugsweise erfolgt die Übertragung der Drehbewegung des eingangsseitigen Getriebegliedes in eine zu seiner Drehachse parallele Translationsbewegung mit Hilfe einer Kurvensteuerung, wobei mindestens ein Teil der Einstich- und bevorzugt auch der Rückführbewegung durch eine Relativbewegung eines Steuerzapfens in einer die Steuerkurve bildenden Ausnehmung bestimmt wird, bei der der Zapfen eine durch die Ausnehmung gebildete Steuerkurve abfährt. Die Ausnehmung, die die Steuerkurve bildet, kann entweder in dem das drehbare Getriebeglied bildenden Bauteil oder in einem benachbarten verschiebbaren Bauteil vorgesehen sein. Entsprechend ist der Zapfen starr mit einem benachbarten verschiebbaren Bauteil bzw. mit dem das drehbare Getriebeglied bildenden Bauteil verbunden.

Bevorzugt ist eine Ausbildung, bei der das drehbare Getriebeglied des Drehschiebegetriebes eine zylindrische Hülse aufweist, innerhalb der ein kolbenförmiges Teil angeordnet ist, das bei Längsverschiebung in Richtung des Einstichweges mit einer zylindrischen Außenwand in der Hülse gleitet, wobei in diesem Falle die Hülse drehbar, jedoch in axialer Richtung nicht verschiebbar und das darin angeordnete axial verschiebbare kolbenförmige Teil drehfest sein sollte. In diesem Falle ist das kolbenförmige Teil vorzugsweise ein Bestandteil des Lanzettenhalters oder mit diesem fest verbunden.

Das erfindungsgemäße Blutentnahmegerät zeichnet sich vor allem dadurch aus, daß die Erschütterungen bei der Einstich- und Rückführbewegung sehr gering sind, weil außer dem Lanzettenhalter selbst keine weiteren Bauteile in der Einstichrichtung beschleunigt und abgebremst werden. Im Rahmen der Erfindung wurde gefunden, daß diese Erschütterungsarmut wesentlich zu einem schmerzarmen Einstich beiträgt.

Weiterhin erlaubt die Erfindung eine einfache und spielarme Konstruktion des Antriebs mit wenigen Bauteilen und guter Führung des Lanzettenhalters. Dadurch werden bei geringen Herstellungskosten Vibrationen bei der Einstich- und Rückführbewegung weitgehend vermieden. Auch dies ist - wie im Rahmen der Erfindung festgestellt wurde - ein wesentlicher Beitrag zur Reduzierung des Schmerzempfindens.

Außerdem erlaubt die Erfindung eine handliche und kompakte Bauweise, wobei das Gehäuse vorzugsweise eine längliche Form (sogenannte "Pencil-Form") hat und seine Längsachse parallel zur Einstichrichtung verläuft. Dies ermöglicht eine bequeme Aufbewahrung und erleichtert die Handhabung.

Gemäß einem zweiten Hauptaspekt der Erfindung sind der Lanzettenantrieb und die Halterung der Lanzette in dem Lanzettenhalter so ausgebildet, daß die Einstichtiefe, um die die Spitze der Lanzette bei der Einstichbewegung aus der Austrittsöffnung hervortritt, bei unveränderter Einstellung der Blutlanzettenvorrichtung und aufeinanderfolgenden Einstichbewegungen um nicht mehr als höchstens 0,15 mm, bevorzugt höchstens 0,1 mm, besonders bevorzugt höchstens 0,05 mm variiert.

Die Einstichtiefe sollte durch den Benutzer selbst leicht und präzise einstellbar sein. Vorzugsweise umfaßt der Einstellbereich dabei ungewöhnlich niedrige Einstichtiefen zwischen 0,5 und 2,0 mm, wobei der Bereich zwischen 0,7 mm und 1,3 mm von besonderer Bedeutung ist. Im Rahmen der vorliegenden Erfindung wurde festgestellt, daß die in der Praxis für die Analyse benötigte Blutmenge, welche üblicherweise zwischen 1 und 50 µl, in der Mehrzahl der Fälle zwischen 10 und 30 µl, liegt, bei der überwiegenden Mehrzahl der Menschen überraschenderweise bereits mit solchen niedrigen Einstichtiefen bei deutlich reduziertem Schmerz gewonnen werden kann, wenn man gleichzeitig dafür sorgt, daß die Tiefe des Einstichs bei einer bestimmten, unveränderten Einstellung des Gerätes außerordentlich gut reproduzierbar ist.

Zwar wird auch bei vorbekannten Blutlanzettenvorrichtungen, beispielsweise der in der US-Patentschrift 4 442 836 beschriebenen, eine reproduzierbare Einstichtiefe angestrebt. Untersuchungen im Rahmen der vorliegenden Erfindung haben jedoch gezeigt, daß bei sämtlichen gebräuchlichen Blutlanzettenvorrichtungen die Schwankungen der Einstichtiefe bei aufeinanderfolgenden Einstichbewegungen wesentlich höher als die vorgenannten Grenzwerte (mindestens etwa ±0,3 mm) sind.

Die Einstellmechanik für die Einstellung der Einstichtiefe ist vorzugsweise so ausgebildet, daß sie stufenweise einstellbar ist, wobei der Stufenabstand zumindest in dem oben genannten bevorzugten Einstellbereich (0,5 bis 2,0 mm, bevorzugt 0,7 bis 1,3 mm) höchstens etwa 0,4 mm und mindestens etwa 0,2 mm, bevorzugt etwa 0,3 mm beträgt. Selbstverständlich kann der gesamte Einstellbereich über die genannten Teilbereiche hinausgehen und auch größere Einstichtiefen umfassen, um den Erfordernissen der relativ wenigen Personen Rechnung zu tragen, bei denen mit den genannten niedrigen Einstichtiefen (beispielsweise wegen einer besonders dicken Hornhaut) keine ausreichende Blutmenge gewonnen werden kann.

Die Reproduzierbarkeit der Einstichtiefe ist vor allem von zwei Faktoren abhängig. Erstens ist wichtig, daß die Bewegung der Lanzettenhalterung derartig präzise gesteuert ist, daß der tiefste Punkt ihrer Bewegung in Einstichrichtung reproduzierbar ist. Zum zweiten kommt es darauf an, daß die Lanzette in ihrer Halterung derartig präzise positioniert ist, daß die Position der Spitze in Richtung der Einstichbewegung relativ zu dem Lanzettenhalter reproduzierbar ist, wenn nacheinander mehrere Lanzetten in der Lanzettenhalterung befestigt werden. Beide Bedingungen lassen sich mit bekannten mechanischen Mitteln ohne weiteres erreichen, sofern bei der Herstellung verhältnismäßig hochwertige Materialien verwendet werden und mit geringen Toleranzen produziert wird. Der im Zusammenhang mit dem ersten Hauptaspekt der Erfindung erläuterte Lanzettenantrieb ist besonders bevorzugt, weil er die gewünschte Reproduzierbarkeit mit relativ geringem Herstellungsaufwand erreicht.

Eine wesentliche Verbesserung der Positionierung der Lanzette in dem Lanzettenhalter und infolgedessen der Reproduzierbarkeit der Einstichtiefe wird durch eine bevorzugte Ausführungsform erreicht, bei der die metallische Nadel der Lanzette ein Positionierungselement und der Lanzettenhalter einen Anschlag für das Positionierungselement aufweist und die Lanzette in dem Lanzettenhalter so gehalten ist, daß das Positionierungselement elastisch gegen den Anschlag gedrückt wird.

Die Lanzetten haben stets eine metallische Nadel, deren eines Ende spitz zugeschliffen ist. Der hintere, von der Spitze abgewandte Teil der Lanzettennadel ist üblicherweise von einem Lanzettenkörper aus Kunststoff umschlossen (US 3,358,689). Die Herstellung erfolgt meist in der Weise, daß die Lanzettennadel in einer Kunststoffspritzform positioniert und der Lanzettenkörper angespritzt wird. Derartige Lanzetten werden mit Hilfe des Lanzettenkörpers in dem Lanzettenhalter fixiert. Die Tiefe der Einstichbewegung wird meist dadurch begrenzt, daß die vordere Kante des Lanzettenkörpers beim Einstich gegen einen mit dem Gehäuse der Lanzettenvorrichtungen starr verbundenen Anschlag im Bereich der Blutlanzettenaustrittsöffnung anschlägt. Dabei wird die Einstichtiefe durch mehrere Toleranzen beeinflußt. Vor allem wurde im Rahmen der vorliegenden Erfindung festgestellt, daß die Fertigungstoleranz der Position der Nadelspitze in Relation zur Position des Kunststoffkörpers ein wesentlicher, die Reproduzierbarkeit beeinträchtigender Faktor ist.

Bei der hier vorgeschlagenen Konstruktion wird die Variation der Einstichtiefe von Lanzette zu Lanzette nur durch die Toleranz des Abstandes zwischen dem Positionierungselement und der Spitze der metallischen Nadel beeinflußt. Das Positionierungselement kann -wie weiter unten anhand von Beispielen erläutert wird- durch das hintere Ende der Nadel oder einen von der Nadel seitlich abstehenden Vorsprung gebildet werden. Dabei ist es mit üblichen Mitteln der Metallverarbeitung ohne weiteres möglich, sehr enge Toleranzen des Abstandes zwischen dem Positionierungselement und der Spitze der Lanzette einzuhalten.

Die Erfindung wird im folgenden anhand eines in den Figuren schematisch dargestellten Ausführungsbeispiels näher erläutert, wobei das Blutentnahmegerät zusammen mit einer darin eingesetzten Lanzette als Blutlanzettenvorrichtung bezeichnet wird; es zeigen:
- Fig. 1: eine Schnittdarstellung einer Blutlanzettenvorrichtung entlang der Längsachse,
- Fig. 2: eine Schnittdarstellung einer Blutlanzettenvorrichtung nach Fig. 1 längs der Linie A-A,

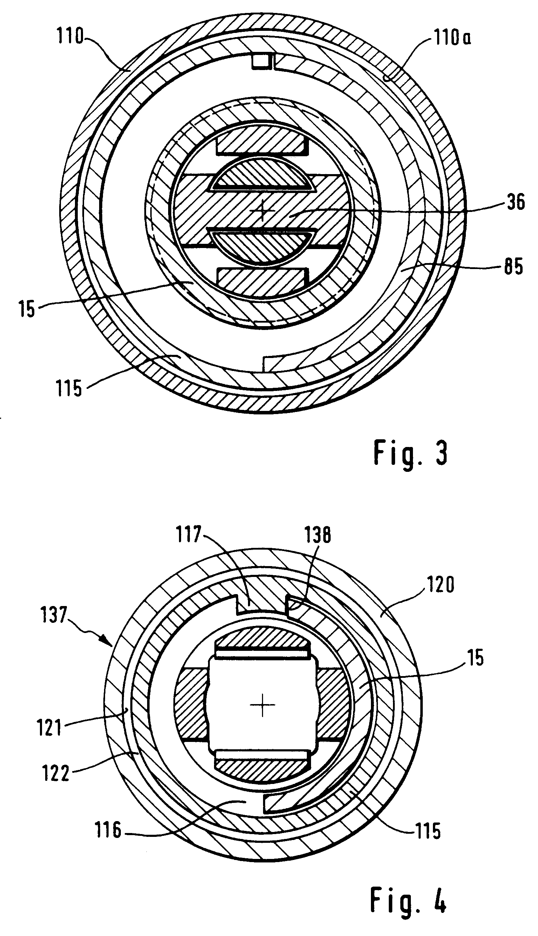

- Fig. 3: eine Schnittdarstellung einer Blutlanzettenvorrichtung nach Fig. 1 längs der Linie B-B,
- Fig. 4: eine Schnittdarstellung einer Blutlanzettenvorrichtung nach Fig. 1 längs der Linie C-C,
- Fig. 5: eine Seitenansicht des Lanzettenhalters der Blutlanzettenvorrichtung nach Fig. 1,
- Fig. 6: eine Projektion der Konturen der Steuerkurvenausnehmung des Lanzettenhalters nach Fig. 5 in eine Ebene,
- Fig. 7: eine teilweise aufgebrochene Darstellung des Stößels und des Lanzettenhalters einer Blutlanzettenvorrichtung nach Fig. 1,
- Fig. 8: eine perspektivische Schnittdarstellung des Gehäusezylinders einer Blutlanzettenvorrichtung nach Fig. 1,
- Fig. 9: eine teilweise aufgebrochene Darstellung von Verschlußhülse und Verschlußkappe einer Blutlanzettenvorrichtung nach Fig. 1,
- Fig. 10: eine Seitenansicht der zylindrischen Hülse einer Blutlanzettenvorrichtung nach Fig. 1,
- Fig. 11: eine bevorzugte Ausführungsform der Lanzettenhalterung und der Lanzette, teilweise in Seitenansicht und teilweise im Schnitt,
- Fig. 12 und Fig. 13: zwei zueinander senkrechte Schnittdarstellungen von einer alternativen bevorzugten Ausführungsform der Lanzettenhalterung und der Lanzette,
- Fig. 14: eine graphische Darstellung zur Erläuterung der Funktion der Erfindung.

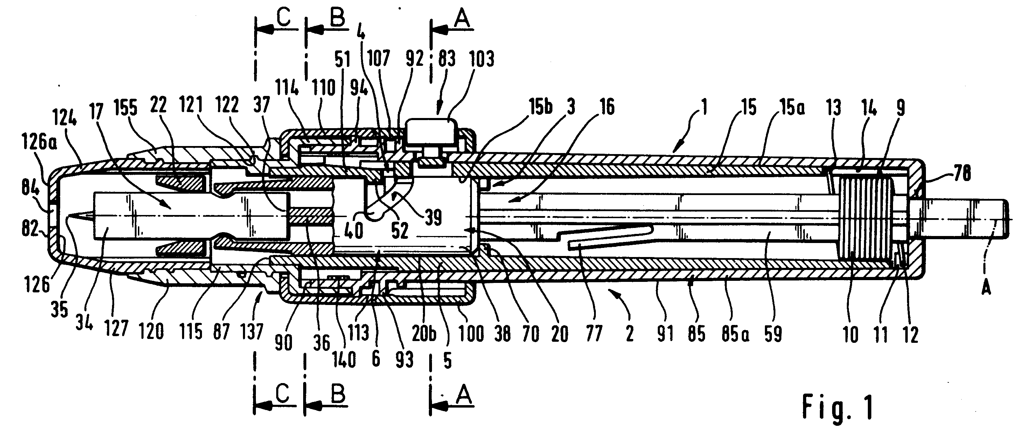

Innerhalb des Gehäuses 1 der in Fig. 1 dargestellten Blutlanzettenvorrichtung 2 ist ein Lanzettenantrieb 3 angeordnet, der ein Drehschiebegetriebe 4 mit einem drehbaren eingangsseitigen Getriebeglied 5 und einem in Richtung der Drehachse A des Getriebegliedes 5 verschiebbaren Lanzettenhalter 6 aufweist. Das an der Eingangsseite 16 des Drehschiebegetriebes 4 eingeleitete Drehmoment wird von einem elastischen Antriebselementes 9 des Lanzettenantriebes 3 erzeugt.

Das elastische Antriebselement 9 ist in der dargestellten bevorzugten Ausführungsform eine schraubenförmig gewundene Biegefeder 10. Diese stützt sich an einem Federanschlag 11 des Gehäuses 1 mit einem Ende 12 ab und mit dem zweiten Ende 13 in einen axialen Schlitz 14 am eingangsseitigen (in der Figur rechten) Ende des Getriebegliedes 5, das als zylindrische Hülse 15 ausgebildet ist. Dabei ist die schraubenförmig gewundene Biegefeder 10 koaxial zu der Drehachse A des Drehschiebegetriebes 4 angeordnet.

Innerhalb der Hülse 15 befindet sich der Lanzettenhalter 6. Ein kolbenförmiges Teil 20, dessen Außendurchmesser etwas kleiner ist als der Innendurchmesser der Hülse 15, ist Bestandteil des Lanzettenhalters 6 und kann mit seiner zylindrischen Außenwand 20b entlang der inneren Wand der Hülse 15 gleiten. Die innere Wand der Hülse 15 bildet somit eine Lanzettenführung 15b bei der Einstich- und Rückführbewegung. Die Hülse 15 ist gegen eine axiale Verschiebung im Gehäuse 1 fixiert und kann nur eine Drehbewegung um die Drehachse A ausführen.

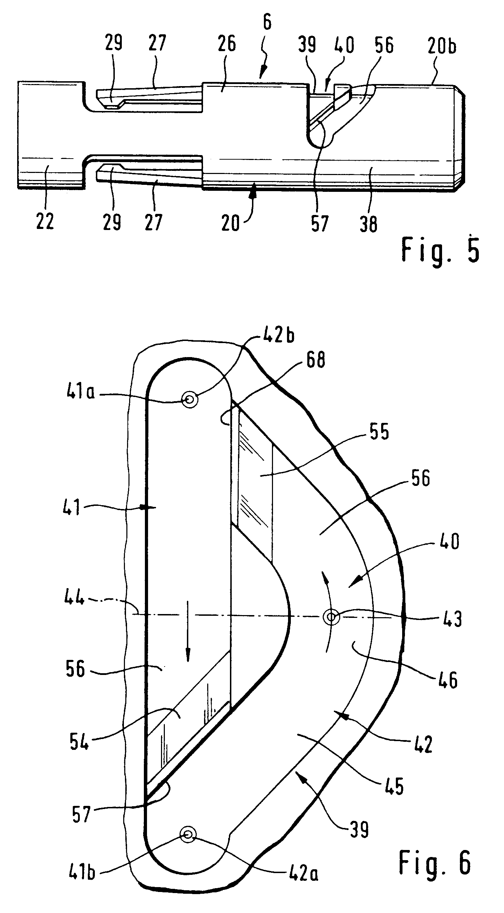

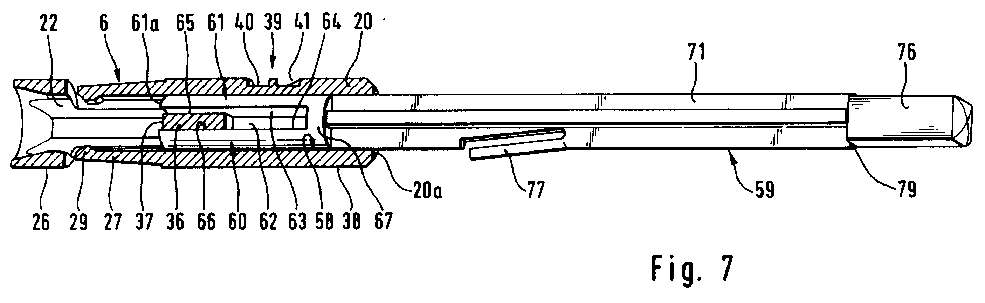

Der Lanzettenhalter 6 ist im ganzen etwa als Hohlzylinder mit näherungsweise konstantem Innendurchmesser ausgebildet (Fig.5 und Fig.7). Im Lanzettenaufnahmeteil 22 ist der Lanzettenhalter 6 mit zwei Ausbrüchen an seiner Mantelwand 26 versehen, in die zwei symmetrische und etwa achsparallel angeordnete Zungen 27 ragen. Diese sind jeweils einseitig mit dem kolbenförmigen Teil 20 verbunden. An den freien Enden der beiden Zungen 27 ist jeweils eine Nase 29 ausgebildet, deren Abstand zueinander im entspannten Zustand der Zungen 27 geringer ist als der Innendurchmesser des Lanzettenhalters 6. Befindet sich eine (in Fig. 1 dargestellte) Lanzette 34 im Lanzettenhalter 6, so halten die Zungen 27 mit den daran ausgebildeten elastisch nach innen gedrückten Nasen 29 die Lanzette 34 zangenartig fest. Das hintere Ende der Lanzette 34 liegt an der Stirnseite 37 eines Steges 36 an, der diametrisch im Lanzettenhalter 6 angeordnet ist.

Wie aus Fig. 1, Fig. 5, Fig. 7 und Fig. 11 ersichtlich ist, ist der Mantel 38 des kolbenförmigen Teiles 20 mit einer nutenförmigen Ausnehmung 39 mit einem rechteckigen Querschnitt versehen, welche eine Steuerkurve 40 für das Drehschiebegetriebe 4 bildet. Die Abwicklung der Konturen der Ausnehmung 39 in eine Ebene ist in Fig. 6 dargestellt. Es ist zu erkennen, daß die Ausnehmung 39 zwei Teilkurven 41,42 mit konstanter Nutenbreite aufweist. Die erste Teilkurve 41 ist entlang einer Umfangslinie des Mantels 38 des zylindrischen Teils 20 ausgebildet, wobei in dem dargestellten bevorzugten Fall der Winkelabstand zwischen dem Anfang 41a und dem Ende 41b der ersten Teilkurve 41 etwa 140° beträgt. Im Bereich des Endes 41b der ersten Teilkurve geht die Ausnehmung 39 in die zweite Teilkurve 42 über, die mit gleicher Nutbreite den Anfang 41a und das Ende 41b der ersten Teilkurve 41 verbindet. Dabei verläuft die zweite Teilkurve 42 bogenförmig. Sie ist ebenso wie die erste Teilkurve 41 symmetrisch bezüglich der Achse 44 angeordnet. Am Anfang 42a weist die zweite Teilkurve 42 ein Geradenstück 45 auf, das stetig in ein Krümmungsstück 46 übergeht, das nach einem Scheitelpunkt 43 symmetrisch zur Achse 44 zu dem Ende 42b läuft. Auch hier entspricht das Ende 42b der zweiten Teilkurve 42 dem Anfang 41a der ersten Teilkurve. Beide Teilkurven 41,42 bilden die ringförmig geschlossene Steuerkurve 40.

An der zylindrischen Hülse 15 (Fig. 1) befindet sich eine etwa achsparallele einseitig befestigte elastische Zunge 51, an deren freiem Ende ein in das Innere der Hülse 15 weisender zylindrischer Steuerzapfen 52 angebracht ist, der in die Ausnehmung 39 des kolbenförmigen Teils 20 eingreift und die durch die Ausnehmung 39 gebildete Steuer-kurve 40 durch eine Relativbewegung mit dem kolbenförmigen Teil abfahren kann. Die Nutbreite der Ausnehmung 39 ist dabei auf den Durchmesser des Zylinders des Steuerzapfens 52 so abgestimmt, daß sich der Steuerzapfen 52 zumindest auf dem größten Teil der Steuerkurve 40 in Formschluß mit der Ausnehmung 39 befindet. Um die Richtung der Relativbewegung zwischen dem Steuerzapfen 52 und der Ausnehmung 39 vorzugeben, ist jeweils vor dem Ende 41b,42b der beiden Teilkurven 41,42 in der Ausnehmung 39 eine rampenartige Stufe 54,55 vorgesehen (Fig.6), die von dem Nutgrund 56 ausgehend gleichmäßig ansteigt um dann mit einer zum Nutgrund 56 senkrecht abfallenden Flanke 57,68 abzuschließen. Dadurch kommt der Steuerzapfen 52 am Ende jeder Teilkurve 41,42 so zu liegen, daß er sich nur in Richtung des Endes 41b,42b der jeweils anderen Teilkurve 41,42 bewegen kann.

In der zylindrischen Ausnehmung 58 des kolbenförmigen Teiles 20 befindet sich ein zylindrisches Teil 60 eines Stößels 59 (Fig.7). Die Längsachse des Stößels 59 stimmt mit der Längsachse des Lanzettenhalters 6 und der Drehachse A der Hülse 15 überein. Das zylindrische Teil 60 des Stößels 59 weist nahezu entlang seiner gesamten Längsachse einen diametrischen Schlitz 62 auf, durch den das zylindrische Teil 60 des Stößels eine Gabel 61 bildet. Der Abstand der beiden planparallelen Flächen 63,64 der Gabel 61 ist auf die Dicke des Steges 36 so abgestimmt, daß das zylindrische Teil 60 des Stößels 59 im kolbenförmigen Teil 20 des Lanzettenhalters 6 axial bewegbar ist, wobei die planparallelen Flächen 63,64 auf den beiden Flächen 65,66 des Steges 36 gleiten. An der geschlossenen Seite 67 der Gabel 61 geht der Stößel 59 in eine Profilstange 71 mit kreuzförmigem Querschnitt über, die so angeordnet sind, daß sie ebenfalls in der zylindrischen Ausnehmung 58 des kolbenförmigen Teils 20 gleiten kann. Am Ende des Stößels 59 befindet sich ein Betätigungsstift 76 mit etwa quadratischem Querschnitt. An der Profilstange 71 des Stößels 59 ist eine Zunge 77 angebracht, die beim Anschlag gegen einen Steg 70 der zylindrischen Hülse 15 die axiale Verschiebung des Stößels 59 im Lanzettenhalter 6 begrenzt.

Der Stößel 59 befindet sich so in der Blutlanzettenvorrichtung 2, daß der Betätigungsstift 76 aus einem entsprechenden Durchbruch 78 des Gehäuses 1 herausragt, wodurch der Stößel 59 gegenüber dem Gehäuse 1 drehfest gelagert ist (Fig.1). Ein am Übergang der Profilstange 71 zu dem Betätigungsknopf ausgebildeter Absatz 79 dient als Anschlag am Gehäuse 1 bei axialer Verschiebung des Stößels 59. Die Profilstange 71 ist durch die schraubenförmige gewundene Biegefeder 10 hindurchgesteckt.

Befindet sich der Steuerzapfen 52 der zylindrischen Hülse 15 am Anfang 41a der ersten Teilkurve 41 der Steuerkurve 40, so ist die Biegefeder 10 in einem ungespannten Zustand (Fig.1 und Fig.6). Durch eine Rechtsdrehung der zylindrischen Hülse 15 wird der Steuerzapfen 52 in der Steuerkurve 40 des kolbenförmigen Teiles 20 zum Ende 41b der ersten Teilkurve 41 bewegt und die mit der Hülse 15 gekoppelte Biegefeder 10 in einen gespannten Zustand gebracht. Dadurch, daß der Stößel 59 in dem Gehäuse 1 drehfest gelagert ist und sich der Steg 36 in der Gabel 61 befindet, wird verhindert, daß sich der Lanzettenhalter 6 bei einer Drehung der Hülse 15 mitdreht. Während dieser Rechtsdrehung der Hülse 15 wird vom Lanzettenhalter 6 keine Längsverschiebung ausgeführt, da die erste Teilkurve 41 entlang einer Umfangslinie des kolbenförmigen Teils 20 verläuft. Am Ende der Stufe 54 befindet sich ein kurzer nicht ansteigender Flächenabschnitt 54a, der etwas tiefer liegt als die Oberkante der Seitenwände der Ausnehmung 39. Die Biegespannung der Zunge 51 bewirkt, daß der Steuerzapfen 52 auf den Nutgrund 56 am Ende der ersten Teilstufe 41b gedrückt wird. Durch die vertikale Flanke 57 der Stufe 54 wird verhindert, daß sich aufgrund des nun gespannten Zustandes der Biegefeder 10 die Hülse 15 mit dem Steuerzapfen 52 entlang der ersten Teilkurve 41 zurückbewegen kann.

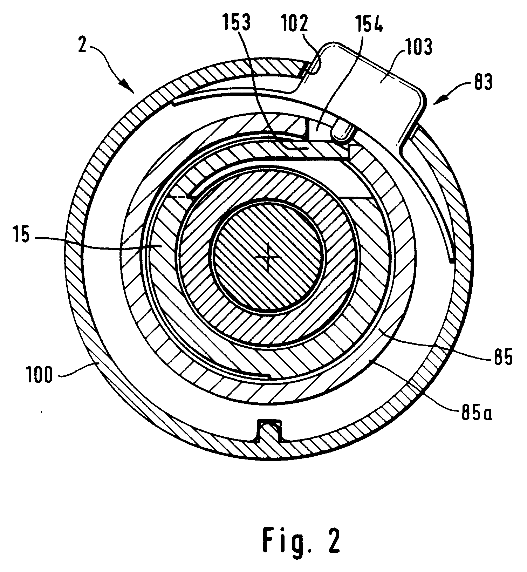

Eine Arretiervorrichtung 83 arretiert die zylindrische Hülse 15 in dieser Position des Steuerzapfens 52 (Fig.1 und Fig.2). Wird die Arretiervorrichtung 83 gelöst, so bewirkt die Federspannung der Biegefeder 10, daß auf die Eingangsseite 16 des Drehschiebegetriebes 4 ein Drehmoment übertragen wird, wobei die Hülse 15 entgegen der vorherigen Drehrichtung zurückgedreht wird und der Steuerzapfen 52 die zweite Teilkurve 42 abfährt (Fig.1 und Fig. 6). Dabei wird an der Ausgangsseite 17 des Drehschiebegetriebes 4 das gegen Drehbewegungen fixierte kolbenförmige Teil 20 im Gehäuse 1 in Richtung auf dessen Austrittsöffnung 84 längsverschoben bis der Steuerzapfen 52 den Scheitel 43 der zweiten Teilkurve 42 erreicht hat und die Spitze 35 der Lanzette 34 durch die Austrittsöffnung 84 austritt. Befindet sich der Steuerzapfen 52 im Scheitel 43 der zweiten Teilkurve 42, so hat die Spitze 35 der Lanzette 34 ihre größte Längsverschiebung in Richtung des Einstichweges erreicht.

Da die Blutlanzettenvorrichtung mit einer die Austrittsöffnung 84 umgebenden Andruckfläche 82 gegen die Haut gedrückt wird, entspricht die Einstichtiefe dem Abstand zwischen der Spitze 35 und der Andruckfläche 82 (in Richtung des Einstichweges).

Die Rückführbewegung der Lanzette 34 ist beendet, wenn der Steuerzapfen das Ende 42b der zweiten Teilkurve 42 erreicht hat, welches identisch ist mit dem Anfang 41a der ersten Teilkurve 41. Kurz bevor er dorthin gelangt, gleitet er über die zweite rampenartige Stufe 55, die ebenfalls einen nicht ansteigenden Flächenabschnitt 55a und eine vertikale Flanke 68 an ihrem Ende aufweist. Die Rampe der Stufe 55 verläuft lang und flach um die Bewegung wenig zu bremsen. Dadurch kann der Steuerzapfen 52 sich nur entlang der ersten Teilkurve 41 weiterbewegen.

Die Einstich- und Rückführbewegung der Lanzette 34 wird durch die Umwandlung der Drehbewegung um die Drehachse A der schraubenförmig gewundenen Biegefeder 10 und des Getriebegliedes 5 in eine Längsverschiebung des Lanzettenhalters 6 in Richtung des vorbestimmten Einstichweges und daran anschließend in die entgegengesetzte Richtung, erreicht.

In einer alternativen Ausführung der Erfindung kann auch vorgesehen sein, daß der Formschluß der Ausnehmung 39 und des Steuerzapfens 52 nur auf Teilabschnitten, insbesondere dem Abschnitt zwischen dem Anfang 41a und dem Scheitel 43 der zweiten Teilkurve 42 gegeben ist.

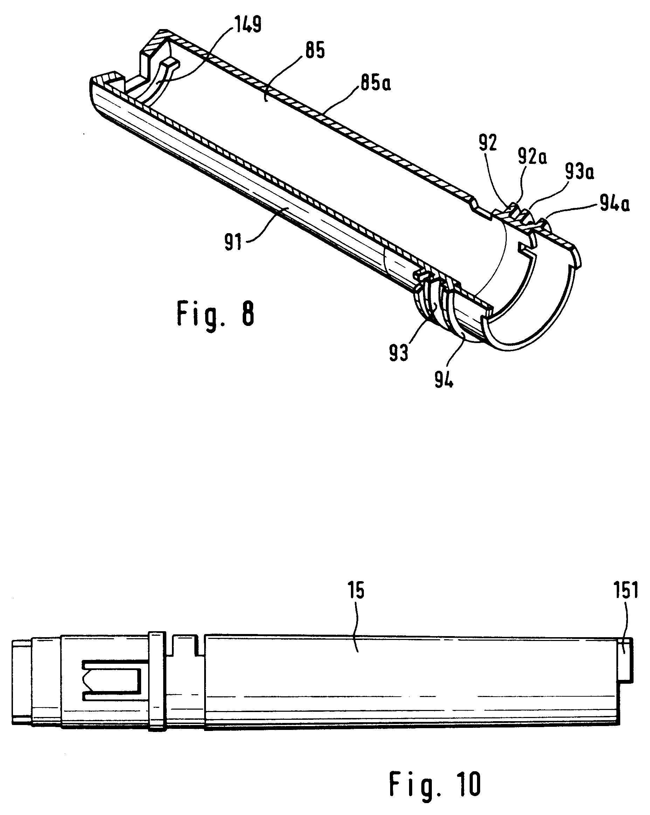

In der in Fig. 1 dargestellten Ausführungsform ist das Gehäuse 1 der Blutlanzettenvorrichtung 2 aus mehreren Elementen zusammengesetzt. Die zylindrische Hülse 15 befindet sich in einem einseitig offenen Gehäusezylinder 85. Dabei ragt die zylindrische Hülse 15 mit ihrer Stirnseite 87 aus dem offenen Ende 90 des Gehäusezylinders 85 heraus. Auf der Mantelfläche 91 des Gehäusezylinders 85 sind im Bereich des offenen Endes 90 drei umlaufende Stege 92,93,94 angebracht (Fig.8), wobei der erste 92 und der zweite Steg 93 mit ihren äußeren Kanten 92a,93a einen größeren Abstand zur Mantelfläche 91 haben als die äußere Kante 94a des dritten Steges 94. Auf den äußeren Kanten 92a,93a dieser zwei Stege 92,93 stützt sich ein Abdeckring 100 ab, der einen Durchbruch 102 für die Aufnahme einer Taste 103 zum Lösen der Arretiervorrichtung 83 aufweist (Fig.2). Der Abdeckring 100 ist an der ersten Stirnseite, die zur Lanzettenspitze 35 weist, offen gestaltet, während die zweite Stirnseite mit einer kreisförmigen Ausnehmung versehen ist, deren Durchmesser etwa dem Durchmesser der Mantelfläche 91 des Gehäusezylinders 85 entspricht. Zur Fixierung am Gehäusezylinder 85 weist der Abdeckring 100 eine Verdrehsicherung und einen an der inneren Mantelfläche in Umfangsrichtung verlaufenden Wulst 107 auf, der sich zwischen dem ersten Steg 92 und zweiten Steg 93 des Gehäusezylinders 85 befindet (Fig.1).

An das offene Ende des Abdeckringes 100 schließt sich bündig ein Zwischenring 110 an, der sich am zweiten Steg 93 des Gehäusezylinders 85 und mit einem innenseitig in Umfangsrichtung verlaufenden Wulst 113 am dritten Steg 94 des Gehäusezylinders 85 abstützt (Fig.1). Der Zwischenring 110 umgreift einen erweiterten Kragen 114 einer Spannhülse 115. Die Spannhülse 115 ist axial etwa in der Position des Lanzettenaufnahmeteils 22 auf die zylindrische Hülse 15 geschoben und mit dieser gegen eine Verschiebung in Richtung der Längsachse fixiert. Dabei ist in dem Mantel der zylindrischen Hülse 15 entlang etwa einer halben Umfangslinie ein Ausbruch 116 vorgesehen, in den ein Mitnehmer 117 der Spannhülse 115 eingreift (Fig.4).

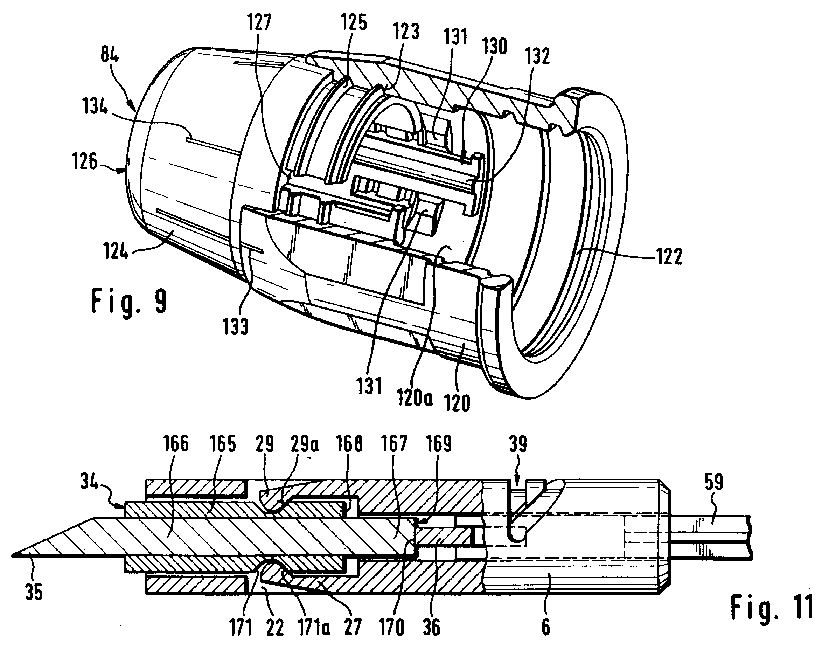

Zum Aufschrauben eines Verschlußringes 120 ist an dem der Austrittsöffnung 84 zugewandten Ende der Spannhülse 115 ein Außengewinde 121 angebracht, während der Verschlußring 120 mit einem entsprechenden Innengewinde 122 versehen ist (Fig.1 und Fig.9). Durch ein zweites Innengewinde 123 ist eine Verschlußkappe 124 mit einem Außengewinde 125 in das der Austrittsöffnung 84 zugewandten Ende des Verschlußrings 120 eingeschraubt. Die Verschlußkappe 124 ist an der ersten Stirnseite 126 abgesehen von der Einstichöffnung 84 geschlossen, wobei die äußere Fläche 126a der Stirnseite 126 bei der Benutzung der Blutlanzettenvorrichtung 2 auf eine Hautstelle gesetzt wird. Zum Einstellen der Einstichtiefe dient das Gewinde 123,125, mit dem die Verschlußkappe 124 und der Verschlußring 120 verbunden sind.

Der Lanzettenantrieb 3 und die Verstellmöglichkeit durch das Gewinde 123,125 sind zusammen mit den Abmessungen der zu verwendenden Lanzetten 34 so abgestimmt, daß die Einstichtiefe in einem gewünschten Einstellbereich, welcher in einer bevorzugten Ausführungsform Einstelltiefen zwischen 0,7 mm und 2,2 mm einschließt, justierbar ist, wobei aufeinanderfolgende Einstichbewegungen bei unveränderter Einstellung der Blutlanzettenvorrichtung 2 bezüglich der Einstichtiefe um höchstens 0,15 mm, bevorzugt höchstens 0,1 mm, besonders bevorzugt höchstens 0,05 mm variieren.

Damit der Benutzer der Blutlanzettenvorrichtung 2 die für sich optimale Einstichtiefe stufenweise verstellen kann und nicht bei jeder Benutzung wieder neu justieren muß, ist eine Einrastvorrichtung 130 vorgesehen, wie Fig. 9 zeigt. Dazu ist der Verschlußring 120 auf seiner inneren Mantelfläche 120a radial umlaufend mit gleichmäßig verteilten erhabenen Noppen 131 versehen. Zwischen jeweils zwei dieser Noppen 131 kommt eine Einrastzunge 132, die an der Verschlußkappe 124 einseitig und parallel zur Längsachse an dem Mantel 127 der Verschlußkappe 124 befestigt ist, nahezu spielfrei in jeweils den freien Bereich zwischen zwei Noppen 131 zu liegen. Durch gegenseitiges Verdrehen von Verschlußkappe 124 und Verschlußring 120 gleitet die elastisch ausgebildete Einrastzunge 132 über einen Noppen 131 hinweg und kommt im nächsten freien Bereich zwischen zwei Noppen 131 zu liegen. Zur Kontrolle der Einstichtiefe ist an dem Verschlußring 120 eine Markierung 133 angebracht, mit deren Hilfe an einer Skalierung 134 an der Verschlußkappe 124 die eingestellte Einstichtiefe zu erkennen ist.

Durch eine Rechtsdrehung des Verschlußringes 120 der Spanneinrichtung 137 gegenüber dem Gehäusezylinder 85 wird die Spannhülse 115 mit der zylindrischen Hülse 15, die mit dem Mitnehmer 117 und der ersten Stirnseite 138 des Ausbruchs 116 im Eingriff stehen, mitgedreht (Fig.1 und Fig. 4). Dadurch wird die Blutlanzettenvorrichtung 2 gespannt. Bei Beginn der Rechtsdrehung steht der Steuerzapfen 52 am Beginn 41a der ersten Teilkurve 41 und fährt diese bis zum Ende 41b ab, wobei die Stufe 55 die Bewegungsrichtung zwangsweise vorgibt. Während dieser Rechtsdrehung verharrt der Lanzettenhalter 6 auf seiner Position im Gehäuse 1 ohne eine Bewegung auszuführen. Dies hat den Vorteil, daß während der Rechtsdrehung die Lanzettenspitze 35 nicht durch die Austrittsöffnung 84 austritt und somit eine ungewollte Verletzungsgefahr während des Spannvorganges ausgeschlossen ist.

Zugleich mit der Rechtsdrehung wird die schraubenförmig gewundenen Biegefeder 10 und ein elastisches Rückführelement, das in der dargestellten bevorzugten Ausführungsform ebenfalls in Form einer schraubenförmig gewundenen Biegefeder 140 ausgebildet ist, in einen gespannten Zustand versetzt. Dabei stützt sich die Rückführfeder 140 einerseits an dem Gehäusezylinder 85 und andererseits an der Spannhülse 115 ab (Fig. 1). Die Rechtsdrehung wird durch das Aufeinandertreffen eines Anschlagsteges 149 des Gehäusezylinders 85 und einer Anschlagnase 151 der zylindrischen Hülse 15 (Fig. 8 und Fig. 10) beendet. Dabei kommt der Steuerzapfen 52 am Ende 41b der ersten Teilkurve 41 hinter der vertikalen Flanke 57 der Stufe 54 zu liegen und eine elastische Arretierungszunge 153 (Fig.2) der Arretiervorrichtung 83 schnappt in eine entsprechende am Mantel 85a des Gehäusezylinders 85 befindliche Arretierungsausnehmung 154 ein und arretiert die Blutlanzettenvorrichtung 2 in dem gespannten Zustand.

Die Spanneinrichtung 137 weist also ein von der Außenseite des Gehäuses 1 zugängliches Betätigungselement 155 (in dem dargestellten bevorzugten Fall den Verschlußring 120) auf, welches mit der Eingangsseite 16 des Drehschiebegetriebes 4 derart gekoppelt ist, daß das elastische Antriebselement 9 des Lanzettenantriebes 3 durch eine Drehung des Betätigungselementes 155 gegenüber dem Gehäuse 1 in den gespannten Zustand gebracht wird (Fig.1).

Beim Loslassen des Verschlußringes 120 kommt die Federspannung der Rückstellfeder 140 zur Wirkung und dreht die miteinander gekoppelten Elemente Spannhülse 115, Verschlußring 120 und Verschlußkappe 124 in ihre Ausgangslage zurück, wobei das Drehschiebegetriebe 4 nicht bewegt wird. Da die Antriebshülse 15 nur bei Rechtsdrehungen des Verschlußringes 120 im Eingriff steht, wird eine Freilauffunktion erreicht. Dadurch, daß das Betätigungselement 155 nach dem Spannen des Antriebselementes 9 in die Ausgangslage zurückgeführt wird, bleibt es bei der Einstichbewegung in Ruhe.

Nach dem Lösen der Arretiervorrichtung 83 durch Drücken der Taste 103, wird der Stechvorgang eingeleitet. Dabei wird der an der Antriebshülse 15 befestigte elastische Arretierungszapfen 153 aus der Arretierungsausnehmung 154 des Gehäusezylinders 85 in den Innenraum des Gehäusezylinders 85 bewegt, wodurch die Antriebshülse 15 für Drehbewegungen gegenüber dem Gehäuse 1 freigegeben ist (Fig.2). Die Lagerung der Taste 103 ist so ausgebildet, daß sie bei ungespannter Blutlanzettenvorrichtung 2 in gedrückter Stellung gehalten wird. Erst im gespannten Zustand der Biegefeder 10 wird die Taste 103 nach außen gedrückt. Damit kann man an der Stellung der Taste 103 den Betriebszustand der Blutlanzettenvorrichtung 2 erkennen. Die Taste 103 wird vom Abdeckring 100 umschlossen und von diesem auch gegenüber dem Gehäuse 1 gehalten.

Die Federspannung der Biegefeder 10 leitet nun eine Linksdrehung der Antriebshülse 15 ein. Während dieser Linksdrehung fährt der Steuerzapfen 52, wie beschrieben, die zweite Teilkurve 42 der Steuerkurve 40 ab.

Dadurch, daß die erfindungsgemäße Blutlanzettenvorrichtung 2 eine geringe beschleunigte Masse in Richtung der Längsbewegung der Lanzette 34 aufweist, ist der Einstich weitestgehend erschütterungsfrei. Dies ist für einen schmerzarmen Einstich eine wichtige Voraussetzung, wie sich in Untersuchungen im Rahmen der Erfindung gezeigt hat. Die beschriebene Führung des Lanzettenhalters 6 innerhalb des Drehschiebegetriebes ist ein weiterer Beitrag zum erschütterungs- und damit auch schmerzfreien Einstich.

Für das Auswechseln der Lanzette 34 wird der Verschlußring 120 mit der Verschlußkappe 124 durch Linksdrehung abgeschraubt. Durch Drücken auf den Betätigungsstift 76 wird der Stößel 59 in Richtung der Austrittsöffnung des Gehäuses entlang der Drehachse A bewegt (Fig.1 und Fig.7). Die Stirnseite 61a der Gabel 61 drückt dabei auf die Lanzette 34, die dadurch ausgeworfen wird. Die axiale Bewegung des Stößels 59 wird durch die Zunge 77 des Stößels begrenzt, die auf den Steg 70 der zylindrischen Hülse 15 trifft. Die neue Lanzette 34 wird in das Lanzettenaufnahmeteil 22 des Lanzettenhalters 6 eingesteckt und bis zur ersten Stirnseite 37 des Steges 36 hineingeschoben. Der Stößel 59 wird dabei zurückgedrückt und die Lanzette 34 durch die beiden Nasen festgeklemmt.

Um eine gute Wiederholgenauigkeit der Einstichtiefe bei unveränderter Einstellung der Blutlanzettenvorrichtung 2 zu erhalten, ist eine exakt reproduzierbare Positionierung der Lanzette 34 im Lanzettenhalter 6 notwendig. Diesem Zweck dienen die in den Figuren 11 bis 13 dargestellten bevorzugten Ausführungsformen der Lanzettenhalterung.

Die in Fig. 11 dargestellte Lanzette 34 besteht wie üblich aus einem Kunststoffkörper 165 und einer metallischen Nadel 166. Sie ist jedoch insofern neuartig ausgebildet, als das hintere Ende 167 der metallischen Nadel 166 über das hintere Ende 168 des Kunststoffkörpers 165 hinausragt. Die rückwärtige Stirnseite des hinteren Endes 167 dient als Positionierungselement 169. Es liegt an einem Anschlag 170 des Lanzettenhalters 7 an, der im dargestellten Fall von dem Steg 36 gebildet wird.

Die Halterung der Lanzette 34 in dem Lanzettenhalter 6 ist so ausgebildet, daß die Lanzette 34 mit dem Positionierungselement 169 gegen den Anschlag 170 nach hinten (entgegen der Einstichrichtung) gedrückt wird. Dies wird in der dargestellten Ausführungsform dadurch erreicht, daß die V-förmigen Ausnehmungen 171, in welche die Nasen 29 der elastischen Zungen 27 eingreifen, so ausgebildet und angeordnet sind, daß die hintere schräge Fläche 29a an einer entsprechenden schrägen Fläche 171a der Ausnehmungen 171 anliegen und aus dem in Richtung auf die Achse A gerichteten Druck der Zungen 27 eine Kraftkomponente entgegen der Einstichrichtung resultiert.

Der Körper 165 der Lanzette 134 ist vorzugsweise nicht rund, sondern beispielsweise quadratisch ausgebildet. Durch eine entsprechende Formgebung des Innenraums des Lanzettenaufnahmeteils 22 wird eine Verdrehsicherung gewährleistet.

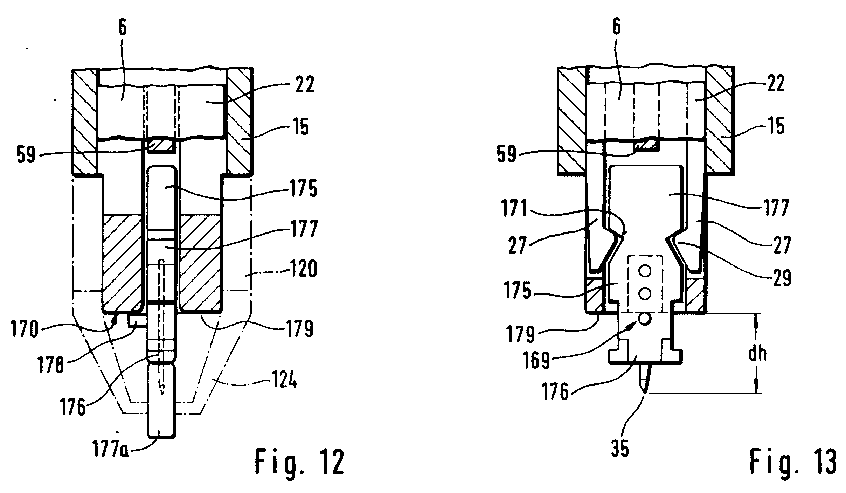

Die in den Figuren 12 und 13 dargestellte Ausführungsform unterscheidet sich vor allem dadurch, daß die Nadel 176 im Querschnitt nicht rund ist, sondern aus einem dünnen Flachmaterial besteht. Die Form der Nadel ist in Fig. 12 in Seitenansicht auf die schmale Kante und in Fig. 13 in Seitenansicht auf die Fläche zu erkennen.

In Fig. 12 ist ein durch Drehen abnehmbarer Spitzenschutz 177a dargestellt, der -wie auch bei anderen Lanzetten üblich- zusammen mit dem Kunststoffkörper 177 an die Nadel 176 angespritzt ist und vor der Benutzung der Lanzette entfernt wird.

Die Nadel 176 ist in einem Kunststoffkörper 177 gefaßt, der auch in diesem Falle V-förmige Ausnehmungen 171 aufweist, die mit den Nasen 29 der elastischen Zungen 27 des Halters 6 in der gleichen Weise wie bei Figur 11 zusammenwirken, um eine resultierende Kraftkomponente zu erzeugen, die auf die Lanzette entgegen der Einstichrichtung wirkt.

Als Positionierungselement 169 dient in diesem Fall ein seitlich von der Nadel 176 abstehender stiftförmiger Vorsprung 178, der an einer den Anschlag 170 bildenden unteren Stirnfläche 179 des Aufnahmeteils 22 des Lanzettenhalters 6 anliegt.

Eine solche Lanzette läßt sich in einem Stanzverfahren einfach herstellen. Sie zeichnet sich durch besonders enge Toleranzen des Abstandes zwischen der Lanzettenspitze 35 und dem Positionierungselement 169 und damit durch eine besonders gute Reproduzierbarkeit der Einstichtiefe aus. Darüberhinaus ermöglicht die flache Form eine in der Ebene der Nadel 176 verhältnismäßig breite Gestaltung der Lanzettenspitze. Dadurch wird schmerzarm mit einer geringen Einstichtiefe eine relativ hohe Blutentnahmemenge erreicht.

Wie weiter oben erwähnt, wurde im Rahmen der vorliegenden Erfindung festgestellt, daß überraschenderweise eine ausreichende Blutmenge mit im Vergleich zum bisher üblichen wesentlich verringerten Einstichtiefen gewonnen werden kann, wenn man gleichzeitig dafür sorgt, daß die Einstichtiefe sehr genau reproduzierbar ist.

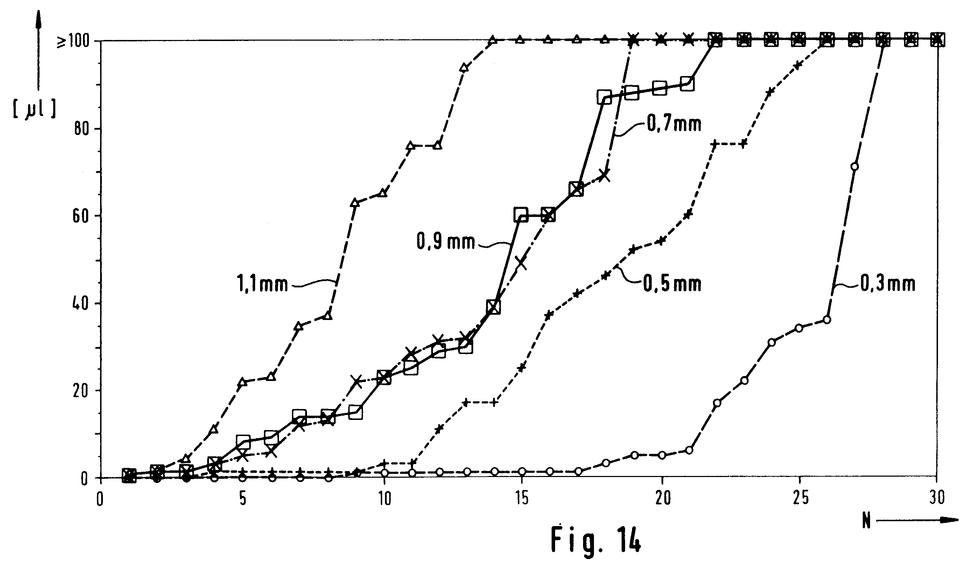

In Fig. 14 sind Ergebnisse, die mit der erfindungsgemäßen Blutlanzettenvorrichtung an dreißig Probanten erzielt wurden, graphisch dargestellt. Dabei ist in der Abszisse die Anzahl N der Probanten und in der Ordinate die gewonnene Blutmenge in µl aufgetragen. Die fünf dargestellten Kurven zeigen die Ergebnisse bei Einstichtiefen von 0,3 mm, 0,5 mm, 0,7 mm, 0,9 mm und 1,1 mm Einstichtiefe. Man erkennt, daß bei 0,3 mm Einstichtiefe bei der überwiegenden Mehrzahl der Probanten unzureichende Blutmengen gewonnen wurden. Bei einundzwanzig Probanten lagen die Blutmengen unter 10 µl.

Bei einer Erhöhung der Einstichtiefe auf 0,5 mm und mehr noch bei einer weiteren Erhöhung auf 0,7 mm oder 0,9 mm nahm die Anzahl der Probanten, bei denen sich eine ausreichende Blutmenge ergab, sehr stark zu. Beispielsweise zeigt Fig. 14, daß bei den Einstichtiefen 0,7 mm und 0,9 mm bereits bei 2/3 der Probanten Blutmengen von 20 µl und mehr gewonnen wurden. Diese Menge reicht bei modernen Analysegeräten vielfach für eine exakte Analyse aus.

Bei einer Einstichtiefe von 1,1 mm nimmt die gewonnene Blutmenge nochmals deutlich zu. Hier liegt sie nur noch bei vier Probanten, also weniger als 15 % der Gesamtzahl der Versuchsteilnehmer unter dem 20µl-Grenzwert.

Berücksichtigt man, daß vorbekannte Blutlanzettenvorrichtungen üblicherweise mit Einstichtiefen über 2 mm arbeiten, um eine ausreichende Blutmenge zu gewinnen, so zeigen die vorstehenden Ergebnisse deutlich, daß bei der großen Mehrzahl der Patienten im Rahmen der Erfindung eine starke Reduzierung des Schmerzes durch Reduzierung der Einstichtiefe erreicht und dennoch eine ausreichende Blutmenge gewonnen werden kann.

## Patentansprüche

1. Blutentnahmegerät zur Entnahme von Blut für Diagnosezwecke, umfassend
ein Gehäuse (1) mit einer Austrittsöffnung (84) für die Spitze (35) der metallischen Nadel (166,176) einer Lanzette (34),
einen in dem Gehäuse (1) entlang einem vorbestimmten Einstichweg beweglichen Lanzettenhalter (6) zur Halterung der Lanzette (34),
eine Lanzettenführung (15b) zur Führung des Lanzettenhalters (6) auf dem vorbestimmten Einstichweg und
einen Lanzettenantrieb (3) mit einem elastischen Antriebselement (9),
der in einer ersten Position, in der sich das elastische Antriebselement (9) in einem gespannten Zustand befindet, mittels einer Arretiervorrichtung (83) arretierbar ist,
durch den nach dem Lösen der Arretiervorrichtung (83) die Entspannungsbewegung des elastischen Antriebselementes (9) in eine Einstichbewegung umgesetzt wird, bei der die von dem Lanzettenhalter (6) gehaltene Lanzette (34) mit hoher Geschwindigkeit entlang dem vorbestimmten Einstichweg in Einstichrichtung bewegt wird, bis ihre Spitze (35) aus der Austrittsöffnung (84) austritt, um in einem an der Austrittsöffnung (84) anliegenden Körperteil eine Wunde zu erzeugen, und
durch den der Lanzettenhalter (6) in eine Position zurückgeführt wird, bei der die Spitze der Lanzette (34) sich in dem Gehäuse befindet,
wobei der Lanzettenantrieb (3) ein Getriebe aufweist, durch welches ein auf der Eingangsseite (16) des Getriebes (4) eingeleitetes Drehmoment in eine Längsverschiebung in Richtung des vorbestimmten Einstichweges umgewandelt wird, wobei das eingangsseitige Getriebeglied (5) des Getriebes (4) mit dem elastischen Antriebselement (9) gekoppelt ist und die ausgangsseitige Längsverschiebung des Getriebes (4) auf den Lanzettenhalter (6) übertragen wird,
**dadurch gekennzeichnet**, daß
das Getriebe ein Drehschiebegetriebe (4) mit einem um eine zu dem vorbestimmten Einstichweg parallele Drehachse (A) drehbaren eingangsseitigen Getriebeglied (5) ist.

2. Blutentnahmegerät nach Anspruch 1, **dadurch gekennzeichnet**, daß das Drehschiebegetriebe (4) eine Kurvensteuerung mit einer eine Steuerkurve (40) bildenden zusammen mit dem eingangsseitigen Getriebeglied (5) drehbaren Ausnehmung (39) aufweist, in die ein passender Steuerzapfen (52) eingreift, wobei mindestens ein Teil der Einstich- und Rückführbewegung durch eine Relativbewegung zwischen dem Steuerzapfen (52) und der Ausnehmung (39) bestimmt wird, bei der der Zapfen (52) die durch die Ausnehmung (39) gebildete Steuerkurve (40) abfährt.

3. Blutentnahmegerät nach einem der vorhergehenden Ansprüche, **dadurch gekennzeichnet**, daß das eingangsseitige Getriebeglied (5) des Drehschiebegetriebes (4) als zylindrische Hülse (15) ausgebildet ist, innerhalb der ein kolbenförmiges Teil (20) angeordnet ist, das bei der Längsverschiebung in Richtung des Einstichweges mit einer zylindrischen Außenwand (20b) in der Hülse (15) gleitet.

4. Blutentnahmegerät nach Anspruch 3, **dadurch gekennzeichnet**, daß das kolbenförmige Teil (20) ein Bestandteil des Lanzettenhalters (6) oder mit diesem fest verbunden ist.

5. Blutentnahmegerät nach einem der vorhergehenden Ansprüche, **dadurch gekennzeichnet**, daß sie eine Spanneinrichtung (137) mit einem von der Außenseite des Gehäuses zugänglichen Betätigungselement (155) aufweist, welches mit dem eingangsseitigen Getriebeglied (5) des Drehschiebegetriebes derartig gekoppelt ist, daß das elastische Antriebselement (9) des Lanzettenantriebs (3) durch Drehung des Betätigungselementes (155) gegenüber dem Gehäuse (1) in den gespannten Zustand gebracht wird.

6. Blutentnahmegerät nach Anspruch 5, **dadurch gekennzeichnet**, daß die Kopplung des Betätigungselementes (155) der Spanneinrichtung (137) mit dem Drehschiebegetriebe (4) einen Freilauf und ein elastisches Rückführelement (140) aufweist, um das Betätigungselement (155) nach dem Spannen des Antriebselementes (9) in die Ausgangslage zurückzuführen.

7. Blutentnahmegerät nach einem der vorhergehenden Ansprüche, **dadurch gekennzeichnet**, daß die Arretiervorrichtung (83) einen Arretierungszapfen (153) aufweist, welcher mit dem eingangsseitigen Getriebeglied (5) des Drehschiebegetriebes (4) fest verbunden ist und im gespannten Zustand des Lanzettenantriebs (3) in eine stationäre Arretierungsausnehmung eingreift.

8. Blutentnahmegerät nach einem der vorhergehenden Ansprüche, **dadurch gekennzeichnet**, daß das elastische Antriebselement (9) eine zu der Drehachse (A) des Drehschiebegetriebes (4) koaxial angeordnete schraubenförmig gewundene Biegefeder (10) ist.

9. Blutentnahmesystem zur Entnahme von Blut für Diagnosezwecke, welches ein Blutentnahmegerät nach einem der vorhergehenden Ansprüche und auf das Blutentnahmegerät abgestimmte Lanzetten umfaßt, wobei der Lanzettenhalter (6) zur auswechselbaren Befestigung einer Lanzette (34) ausgebildet ist und die Lanzetten einen Lanzettenkörper (165) aus Kunststoff und eine darin eingebettete metallische Nadel (166) aufweisen, **dadurch gekennzeichnet,** daß die Lanzette (34) in dem Lanzettenhalter (6) derartig präzise positioniert ist und der Lanzettenantrieb (3) den Lanzettenhalter (6) derartig präzise bewegt, daß die Einstichtiefe, um die die Spitze (35) der Lanzette (34,175) bei der Einstichbewegung aus der Austrittsöffnung (84) austritt, bei unveränderter Einstellung des Blutentnahmegerätes und aufeinanderfolgenden Einstichbewegungen um nicht mehr als höchstens ±0,15 mm, vorzugsweise höchstens ±0,10 mm, besonders bevorzugt höchstens ±0,05 mm, variiert, wenn nacheinander mehrere Lanzetten (34) in dem Lanzettenhalter (6) befestigt werden.

10. Blutentnahmesystem nach Anspruch 9, **dadurch gekennzeichnet**, daß zur präzisen Positionierung der Lanzette (34, 175) die metallische Nadel (166,176) der Lanzette (34,175) ein Positionierungselement (169) und der Lanzettenhalter (6) einen Anschlag (170) für das Positionierungselement (169) aufweist und die Lanzette (34,175) in dem Lanzettenhalter (6) so gehaltert ist, daß das Positionierungselement (169) elastisch gegen den Anschlag (170) gedrückt wird.

11. Blutentnahmesystem nach Anspruch 10, **dadurch gekennzeichnet**, daß das hintere Ende (167) der metallischen Nadel (166) über das hintere Ende (168) des Kunststoffkörpers (165) derartig hinausragt, daß die rückwärtige Stirnseite des hinteren Endes (167) der metallischen Nadel (166) als Positionierungselement (169) gegen den Anschlag (170) des Halters (6) gedrückt wird.

12. Blutentnahmesystem nach einem der Ansprüche 9 bis 11, **dadurch gekennzeichnet**, daß der Kunststoffkörper (165) Ausnehmungen (171) aufweist, in welche elastische Zungen (27) des Lanzettenhalters (6) mit Nasen (29) eingreifen, wobei die Ausnehmungen so ausgebildet und angeordnet sind, daß aus dem in Richtung auf die Drehachse (A) des eingangsseitigen Getriebegliedes (5) des Blutentnahmegerätes gerichteten Druck der Zungen (27) eine Kraftkomponente entgegen der Einstichrichtung resultiert, durch die die Lanzette (34) gegen den Anschlag (170) gedrückt wird.

13. Blutentnahmesystem nach einem der Ansprüche 9 bis 12, **dadurch gekennzeichnet,** daß die Lanzette (34) und der Innenraum des Lanzettenaufnahmeteils (22) des Lanzettenhalters (6) eine einander entsprechende nichtrunde Formgebung haben, um eine Verdrehsicherung zu gewährleisten.

14. Blutentnahmesystem nach einem der Ansprüche 9 bis 13, **dadurch gekennzeichnet**, daß das Blutentnahmegerät eine Einstellmechanik zur Einstellung der Einstichtiefe aufweist und die Abmessungen der Lanzetten (34) und die Einstellmechanik zur Einstellung der Einstichtiefe so aufeinander abgestimmt sind, daß die Einstichtiefe in einem Einstellbereich einstellbar ist, welcher Einstellwerte unterhalb 2 mm, vorzugsweise unterhalb 1,3 mm einschließt.

15. Blutentnahmesystem nach Anspruch 14, **dadurch gekennzeichnet,** daß die Einstellmechanik zwei zusammenwirkende Gewinde (123, 125) aufweist, um die Einstichtiefe durch eine Drehbewegung leicht und präzise zu justieren.

16. Blutentnahmesystem nach einem der Ansprüche 14 oder 15, **dadurch gekennzeichnet,** daß die Einstichtiefe stufenweise einstellbar ist, wobei der Stufenabstand höchstens 0,4 mm beträgt.

17. Verfahren zur Entnahme einer Blutprobe für Diagnosezwecke aus einem Körperteil unter Erzeugung einer Stichwunde
mittels eines Blutentnahmesystems nach einem der Ansprüche 10 bis 16,
**dadurch gekennzeichnet,** daß man
in den Lanzettenhalter (6) eines Blutentnahmegerätes nach einem der Ansprüche 1 bis 8 eine Lanzette (34) mit einem Lanzettenkörper (165) aus Kunststoff und einer darin eingebetteten metallischen Nadel (166) einsetzt, die auf das Blutentnahmegerät abgestimmt ist und mit diesem ein Blutentnahmesystem bildet, wobei die Lanzette (34) derartig in den Lanzettenhalter (6) eingeführt wird, daß ein an der metallischen Nadel (166, 176) der Lanzette (34, 175) vorhandenes, aus dem Kunststoffkörper (165) der Lanzette hervorstehendes Positionierungselement (169) elastisch gegen den Anschlag (170) des Lanzettenhalters (106) gedrückt wird, so daß die Lanzette (34) in dem Lanzettenhalter (6) präzise positioniert ist, und
eine die Lanzettenaustrittsöffnung (84) des Blutentnahmegerätes umgebende Andruckfläche (82) gegen die Haut des Patienten drückt und durch Lösen der Arretiervorrichtung (83) die Einstichbewegung des Lanzettenhalters (6) auslöst, wobei die Bewegung des Lanzettenhalters (6) bei der Einstich- und Rückführbewegung derart präzise gesteuert wird, daß die Einstichtiefe, um die die Spitze (35) der Lanzette (34,175) bei der Einstichbewegung aus der Austrittsöffnung (84) austritt, bei unveränderter Einstellung des Blutentnahmegerätes und aufeinanderfolgenden Einstichbewegungen um nicht mehr als höchstens ±0,15 mm, vorzugsweise höchstens ±0,10 mm, besonders bevorzugt höchstens ±0,05 mm, variiert, wenn nacheinander mehrere Lanzetten (34) in dem Lanzettenhalter (6) befestigt werden.

## Claims

1. Blood withdrawal instrument for withdrawing blood for diagnostic purposes, comprising
a housing (1) with an outlet (84) for the tip (35) of the metal needle (166, 176) of a lancet (34),
a lancet holder (6) for holding the lancet (34) and moveable within the housing (1) along a predetermined puncture path,
a lancet guide (15b) for guiding the lancet holder (6) on the predetermined puncture path, and
a lancet drive (3) with an elastic drive element (9),
said lancet drive (3) being lockable by means of a locking device (83) in a first position, in which the elastic drive element (9) is in a tensioned state,
via which lancet drive (3), after the release of the locking device (83), the tension-release movement of the elastic drive element (9) is converted into a puncturing movement, during which the lancet (34) held by the lancet holder (6) is moved at high speed along the predetermined puncture path in a puncturing direction until its tip (35) emerges from the outlet (84), in order to produce a wound in a body part adjoining the outlet (84), and
via which lancet drive (3) the lancet holder (6) is retracted into a position in which the tip of the lancet (34) is positioned within the housing,
wherein the lancet drive (3) comprise a transmission system (4) via which a torque introduced on the input side (16) of the transmission system (4) is converted into a longitudinal displacement in the direction of the predetermined puncture path, the input-side transmission member (5) of the transmission system (4) being coupled with the elastic drive element (9), and the output-side longitudinal displacement of the transmission system (4) being transmitted to the lancet holder (6),
**characterised in that**
the transmission system is a rotary/sliding transmission system (4) with an input-side transmission member (5) rotatable about an axis of rotation (A) parallel to the predetermined puncture path.

2. Blood withdrawal instrument according to claim 1, **characterised in that** the rotary/sliding transmission system (4) has a cam drive mechanism with a rotatable recess (39) forming a driver cam (40), together with the input-side transmission member (5) with which recess a suitable driver pin (52) engages, at least a part of the puncturing and retraction movement being determined by a relative movement between the driver pin (52) and the recess (39), the driver pin (52) travelling along the driver cam (40) formed by the recess (39).

3. Blood withdrawal instrument according to one of the preceding claims, **characterised in that** the input-side transmission member (5) of the rotary/sliding transmission system (4) is in the form of a cylindrical sleeve (15) within which is located a piston-shaped part (20) which, during the longitudinal displacement in the direction of the puncture path, slides with a cylindrical outer wall (20b) within the sleeve (15).

4. Blood withdrawal instrument according to claim 3, **characterised in that** the piston-shaped part (20) is a component part of the lancet holder (6) or firmly connected to it.

5. Blood withdrawal instrument according to one of the preceding claims, **characterised in that** it has a tensioning device (137) with an operating element (155) accessible from the outside of the housing, said operating element being coupled with the input-side transmission member (5) of the rotary/sliding transmission system in such a way that the elastic drive element (9) of the lancet drive (3) can be brought into the tensioned state via rotation of the operating element (155) with respect to the housing (1).

6. Blood withdrawal instrument according to claim 5, **characterised in that** the coupling of the operating element (155) of the tensioning device (137) with the rotary/sliding transmission system (4) has a freewheeling mode and an elastic return element (140), in order to return the operating element (155) into the starting position after the tensioning of the drive element (9).

7. Blood withdrawal instrument according to one of the preceding claims, **characterised in that** the locking device (83) has a locking pin (153) which is firmly connected with the input-side transmission member (5) of the rotary/sliding transmission system (4) and in the tensioned state of the lancet drive (3) engages with a stationary locking recess.

8. Blood withdrawal instrument according to one of the preceding claims, **characterised in that** the elastic drive element (9) is a spirally wound coiled spring (10) located coaxially to the axis of rotation (A) of the rotary/sliding transmission system (4).

9. Blood withdrawal system for withdrawing blood for diagnostic purposes, comprising a blood withdrawal instrument according to any one of the preceding claims and lancets adapted for use with the blood withdrawal instrument, wherein the lancet holder (6) is adapted for exchangibly holding a lancet (34) and the lancets comprise a lancet body (165) of plastic material and a metal needle (166) embedded therein,
**characterised in that** the positioning of the lancet (34) in the lancet holder (6) is so precise and the lancet drive (3) moves the lancet holder (6) so precisely that the puncture depth at which during the puncturing movement the tip (35) of the lancet (34, 175) emerges from the outlet (84) varies by no more than at most ± 0.15 mm, preferably by at most ± 0.10 mm, especially preferably by at most ± 0.05 mm with unchanged setting of the blood withdrawal instrument and successive puncturing movements, when a plurality of lancets (34) are secured to the lancet holder (6) one after the other.

10. Blood withdrawal system according to claim 9 **characterised in that** for the precise positioning of the lancet (34, 175) the metal needle (166, 176) of the lancet (34, 175) has a positioning element (169), the lancet holder (6) has a stop (170) for the positioning element (169), and the lancet (34, 175) is held in the lancet holder (6) in such a way that the positioning element (169) is elastically pressed against the stop (170).

11. Blood withdrawal system according to claim 10, **characterised in that** the posterior end (167) of the metal needle (166) projects beyond the posterior end (168) of the plastic body (165) in such a way that the rearward face of the posterior end (167) of the metal needle (166) is pressed as positioning element (169) against the stop (170) of the holder (6).

12. Blood withdrawal system according to any one of claims 9 to 11, **characterised in that** the plastic body (165) has recesses (171) with which elastic tongues (27) of the lancet holder (6) engage via lugs (29), the recesses being designed and disposed in such a way that a force component in opposition to the puncturing direction results from the pressure of the tongues (27) in the direction of the axis of rotation (A) of the input-side transmission member (5), by which force component the lancet (34) is pressed against the stop (170).

13. Blood withdrawal system according to any one of claims 9 to 12, **characterised in that** the interior space of the lancet take-up part (22) of the lancet holder (6) and the lancet (34) have a mutually corresponding non-round shape to secure against torsion.

14. Blood withdrawal system according to any one of claims 9 to 13, **characterised in that** the blood withdrawal instrument has a setting mechanism for adjusting the puncture depth and the dimensions of the lancets (34) and the setting mechanism for adjusting the puncture depth are mutually adapted in such a way that the puncture depth is adjustable in an adjustement range which includes adjustment values of less than 2 mm, preferably less than 1.3 mm.

15. Blood withdrawal system according to claim 14, **characterised in that** the setting mechanism comprises two cooperating threads (123,125) for easy and precise adjustment of the puncture depth by a rotary movement.

16. Blood withdrawal system according to any one of claims 14 or 15, **characterised in that** the puncture depth is adjustable stepwise, the interval between steps being at least 0.2 mm and at most 0.4 mm.

17. Method for withdrawing a blood sample for diagnostic purposes from a body part while generating a puncture wound
by means of a blood withdrawal system according to any one of claims 10 to 16,
**characterised in that**
a lancet (34) having a plastic lancet body (165) and a metal needle (166) embedded therein is placed in the lancet holder (6) of a blood withdrawal instrument according to any one of claims 1 to 8, the lancet (34) being adapted to the blood withdrawal instrument and forming a blood withdrawal system therewith, wherein the lancet (34) is inserted in the lancet holder (6) in such a way that a positioning element (169) which is provided at the metallic needle (166, 176) of the lancet (34, 175) and protrudes from the plastic body (165) of the lancet is pressed elastically against a stop (170) of the lancet holder (106) whereby the lancet (34) is precisely positioned in the lancet holder (6), and
a contact surface (82) surrounding the lancet outlet (84) of the blood withdrawal instrument is pressed against the skin of the patient and the puncturing movement of the lancet holder (6) is initiated by releasing the locking device (83), wherein the movement of the lancet holder (6) during the puncturing and retraction movement is so precise that the puncture depth, by which the tip (35) of the lancet (34, 175) emerges from the outlet (84) during the puncturing movement varies by no more than at most ± 0.15 mm, preferably by at most ± 0.10 mm, especially preferably by at most ± 0.05 mm with unchanged setting of the blood withdrawal instrument and successive puncturing movements, when a plurality of lancets (34) are secured to the lancet holder (6) one after the other.

## Revendications

1. Dispositif de prélèvement de sang à des fins de diagnostic, comprenant
un boitier (1) comportant un orifice de sortie (84) pour la pointe (35) de l'aiguille métallique (166, 176) d'une lancette (34),
un support de lancette (6), déplaçable lelong d'un trajet de piqûre prédéterminé à l'intérieur du boitier (1), destiné à porter la lancette (34),
un guidage (15b) de lancette destiné à guider le support de lancette (6) suivant le trajet de piqûre prédéterminé et
un propulseur de lancette (3) muni d'un élément propulseur élastique (9),
qui, dans une première position dans laquelle l'élément propulseur élastique (9) se trouve à l'état tendu, peut être arrêté au moyen d'un dispositif d'arrêt (83),
par l'intermédiaire duquel, après la libération du dispositif d'arrêt (83), la détente de l'élément propulseur élastique (9) est transformée en un déplacement de piqûre, au cours duquel la lancette (34), maintenue par le support de lancette (6), est déplacée très rapidement lelong du trajet de piqûre prédéterminé, dans le sens de la piqûre, jusqu'à ce que sa pointe (35) sorte par l'orifice de sortie (84), pour produire une blessure sur une partie corporelle reposant sur l'orifice de sortie (84), et
par l'intermédiaire du support de lancette (6), il est ramené dans une position dans laquelle la pointe de la lancette (34) se trouve dans le boitier,
dans lequel le propulseur de lancette (3) présente un organe de transmission par l'intermédiaire duquel un couple de rotation, induit sur la face d'entrée (16) de l'organe de transmission (4), est transformé en un déplacement longitudinal dans le sens du trajet de piqûre prédéterminé, l'élément de transmission (5) côté entrée de l'organe de transmission (4) étant couplé à l'élément propulseur élastique (9) et le déplacement longitudinal côté sortie de l'organe de transmission (4) étant transmis au support de lancette (6),
caractérisé en ce que l'organe de transmission est un organe de transmission (4) de mouvement rotatif avec un élément de transmission (5) côté entrée tournant autour d'un axe de rotation (A) parallèle au trajet de piqûre prédéterminé.

2. Dispositif de prélèvement de sang selon la revendication 1, caractérisé en ce que l'organe de transmission (4) de mouvement rotatif présente une commande curviligne comportant une cavité (39) formant une courbe de commande (40), tournant avec l'élément de transmission (5) côté entrée, dans laquelle vient s'enclencher un bouton de commande (52) de forme adaptée, au moins une partie du déplacement de piqûre et du déplacement de retour étant déterminée par un déplacement relatif entre le bouton de commande (52) et la cavité (39), dans lequel le bouton (52) parcourt la courbe de commande (40) formée par la cavité (39).

3. Dispositif de prélèvement de sang selon l'une quelconque des revendications précédentes, caractérisé en ce que l'élément de transmission (5) côté entrée de l'organe de transmission (4) de mouvement rotatif est conçu sous la forme d'une douille cylindrique (15), à l'intérieur de laquelle est disposée une pièce en forme de piston (20) qui coulisse à l'intérieur de la douille (15), avec une paroi extérieure cylindrique (20b), pendant le déplacement longitudinal dans le sens du trajet de piqûre.

4. Dispositif de prélèvement de sang selon la revendication 3, caractérisé en ce que la pièce en forme de piston (20) est une partie constitutive du support de lancette (6) ou est fermement reliée à celui-ci.

5. Dispositif de prélèvement de sang selon l'une quelconque des revendications précédentes, caractérisé en ce qu'il présente un dispositif tendeur (137) avec un élément de commande (155) accessible par la face extérieure du boitier, qui est couplé à l'élément de transmission côté entrée (5) de l'organe de transmission de mouvement rotatif de telle façon que l'élément propulseur (9) élastique du propulseur de lancette (3) soit mis sous tension par rotation de l'élément de commande (155) par rapport au boitier (1).

6. Dispositif de prélèvement de sang selon la revendication 5, caractérisé en ce que le couplage de l'élément de commande (155) du dispositif tendeur (137) avec l'organe de transmission de mouvement rotatif (4) présente une roue libre et un élément de retour élastique (140) pour ramener l'élément de commande (155), après la tension de l'élément propulseur élastique (9), dans la position initiale.

7. Dispositif de prélèvement de sang selon l'une quelconque des revendications précédentes, caractérisé en ce que le dispositif d'arrêt (83) présente un bouton d'arrêt (153) qui est relié fermement à l'élément de transmission (5) côté entrée de l'organe de transmission de mouvement rotatif (4) et qui est enclenché dans un orifice d'arrêt fixe pendant que le propulseur de lancette (3) est sous tension.

8. Dispositif de prélèvement de sang selon l'une quelconque des revendications précédentes, caractérisé en ce que l'élément propulseur (9) élastique est un ressort de flexion (10) hélicoïdal, disposé coaxialement par rapport à l'axe de rotation (A) de l'organe de transmission (4) de mouvement rotatif.

9. Système de prélèvement de sang à des fins de diagnostic, comprenant un dispositif de prélèvement de sang selon l'une quelconque des revendications précédentes, et des lancettes adaptées à ce dispositif de prélèvement de sang, le support de lancette (6) étant conçu pour la fixation d'une lancette (34) de manière rechargeable et les lancettes présentant un corps de lancette (165) en matière plastique et une aiguille métallique (166) incorporée dans celui-ci,
caractérisé en ce que la lancette (34) est positionnée dans le support de lancette (6) avec une précision telle, et le propulseur de lancette (3) déplace le support de lancette (6) avec une précision telle, que la profondeur de piqûre, correspondant à la longueur de la pointe (35) de la lancette (34,175) qui dépasse de l'orifice de sortie (84) pendant le déplacement de piqûre, pour un réglage inchangé du dispositif de prélèvement de sang et pendant des déplacements de piqûre consécutifs, ne varie au plus que de ± 0,15 mm, de préférence de ± 0,10 mm, de manière particulièrement préférée, de ± 0,05 mm, lorsque plusieurs lancettes (34) sont fixées les unes après les autres dans le support de lancette (6).

10. Système de prélèvement de sang selon la revendication 9, caractérisé en ce que pour le positionnement précis de la lancette (34,175), l'aiguille métallique (166,176) de la lancette (34,175) présente un élément de positionnement (169) et le support de lancette (6) présente une butée (170) pour l'élément de positionnement (169) et la lancette (34,175) est maintenue dans le support de lancette (6) de façon que l'élément de positionnement (169) soit soumis à une poussée élastique contre la butée (170).

11. Système de prélèvement de sang selon la revendication 10, caractérisé en ce que l'extrémité arrière (167) de l'aiguille métallique (166) dépasse l'extrémité arrière (168) du corps en matière plastique de telle façon, que la face frontale arrière de l'extrémité arrière (167) de l'aiguille métallique (166) soit poussée, en tant qu'élément de positionnement (169), contre la butée (170) du support (6).

12. Système de prélèvement de sang selon l'une quelconque des revendications 9 à 11, caractérisé en ce que le corps en matière plastique (165) présente des cavités (171) dans lesquelles viennent s'enclencher des pattes élastiques (27) du support de lancette (6) avec des nez (29), les cavités étant conçues et disposées de telle façon que la pression des pattes (27) dirigée vers l'axe de rotation (A) de l'élément de transmission (5) côté entrée du dispositif de prélèvement de sang produise une composante de force dirigée contre le sens de la piqûre, qui pousse la lancette (34) contre la butée (170).

13. Système de prélèvement de sang selon l'une quelconque des revendications 9 à 12, caractérisé en ce que la lancette (34) et l'espace intérieur de la partie de réception de lancette (22) du support de lancette (6) présentent des formes non arrondies correspondantes, pour assurer une fixation contre la torsion.

14. Système de prélèvement de sang selon l'une quelconque des revendications 9 à 13, caractérisé en ce que le dispositif de prélèvement de sang présente un mécanisme de réglage pour le réglage de la profondeur de piqûre et que les dimensions de la lancette (34) et le mécanisme de réglage pour le réglage de la profondeur de piqûre sont ajustées entre elles de façon que la profondeur de piqûre soit réglable à l'intérieur d'une gamme de réglage qui comprend des valeurs de réglage inférieures à 2 mm, de préférence inférieures à 1,3 mm.

15. Système de prélèvement de sang selon la revendication 14, caractérisé en ce que le mécanisme de réglage présente deux filetages (123,125) coopérant entre eux, pour ajuster la profondeur de piqûre facilement et avec précision par un mouvement rotatif.

16. Système de prélèvement de sang selon l'une quelconque des revendications 14 ou 15, caractérisé en ce que la profondeur de piqûre est réglable par paliers, la hauteur de palier étant d'au plus 0,4 mm.

17. Procédé de prélèvement d'un échantillon de sang à des fins de diagnostic à partir d'une partie corporelle en produisant une blessure en forme de piqûre, à l'aide d'un système de prélèvement de sang selon l'une quelconque des revendications 10 à 16,
caractérisé en ce que l'on dispose, dans le support de lancette (6) d'un dispositif de prélèvement de sang selon l'une quelconque des revendications 1 à 8, une lancette (34) comprenant un corps de lancette (165) en matière plastique et une aiguille métallique (166) incorporée dans celui-ci, qui est adaptée au dispositif de prélèvement de sang et forme avec celui-ci un système de prélèvement de sang, la lancette (34) étant introduite dans le support de lancette (6) de telle façon, qu'un élément de positionnement (169) disposé sur l'aiguille métallique (166,176) de la lancette (34,175), faisant saillie du corps en matière plastique (165) de la lancette, soit poussé de manière élastique contre la butée (170) du support de lancette (106), de sorte que la lancette (34) soit positionnée avec précision dans le support de lancette (6), et
une surface de pression (82) entourant l'orifice de sortie de lancette (84) du dispositif de prélèvement de sang est poussée contre la peau du patient et par libération du dispositif d'arrêt (83), déclenche la translation de piqûre du support de lancette (6), le déplacement du support de lancette (6) pendant le déplacement de piqûre et pendant le déplacement de retour est commandé avec une précision telle que la profondeur de piqûre, correspondant à la longueur de la pointe (35) de la lancette (34,175) qui dépasse de l'orifice de sortie (84) pendant le déplacement de piqûre, pour un réglage inchangé du dispositif de prélèvement de sang et pendant des déplacements de piqûre consécutifs, ne varie au plus que de ± 0,15 mm, de préférence de ± 0,10 mm, de manière particulièrement préférée, de ± 0,05 mm, lorsque plusieurs lancettes (34) sont fixées les unes après les autres dans le support de lancette (6).
